Europäisches Patentamt

European Patent Office

Office européen des brevets

(11) Veröffentlichungsnummer: **0 237 620 B1**

## (12) EUROPÄISCHE PATENTSCHRIFT

(45) Veröffentlichungstag der Patentschrift:
22.08.90

(21) Anmeldenummer: 86114246.1

(22) Anmeldetag: 15.10.86

(51) Int. Cl.⁵: **B01D 61/42, C12P 7/40,**
C12P 7/46, C25B 3/00

(54) Verfahren zur Gewinnung von organischen Säuren.

(30) Priorität: 04.12.85 DE 3542861

(43) Veröffentlichungstag der Anmeldung:
23.09.87 Patentblatt 87/39

(45) Bekanntmachung des Hinweises auf die Patenterteilung:
22.08.90 Patentblatt 90/34

(84) Benannte Vertragsstaaten:
AT BE CH DE FR GB IT LI LU NL SE

(56) Entgegenhaltungen:
DE-A- 1 251 732
DE-A- 1 417 033
DE-B- 1 054 068
GB-A- 1 493 866
GB-A- 2 131 049
US-A- 4 282 323

(73) Patentinhaber: HÜLS AKTIENGESELLSCHAFT,
Patentabteilung / PB 15 - Postfach 13 20,
D-4370 Marl 1(DE)

(72) Erfinder: Sridhar, Srinivasan, Dr., Lehmbecker Pfad 52,
D-4370 Marl(DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein neues Verfahren zur Gewinnung einer freien organischen Säure aus einem Salz dieser Säure durch Elektrodialyse, bei der das Salz der organischen Säure in die freie organische Säure übergeführt wird und gleichzeitig das Kation des Salzes der organischen Säure mit dem Anion einer anderen Säure ein Salz bildet. Beide Umwandlungen werden dabei in ein- und derselben Elektrodialyseeinheit ausgeführt.

Für die fermentative Umsetzung von zum Beispiel Fumarsäure zu L-Äpfelsäure können Bakterien (DE-B 2 363 285), immobilisierte Bakterien (Europ. J. Appl. Microbiol. 3 (1976), 169), Pilze (DE-A 3 310 849) oder auch immobilisierte Pilze (DE-A 2 415 310) benutzt werden. L-Äpfelsäure kann auch mit Hilfe von Pilzen und Bakterien aus Glucose hergestellt werden (J. Ferment. Technol. 54 (1976) 197).

Bei der Aufarbeitung von fermentativ hergestellten organischen Säuren wird die Methode der Elektrodialyse entweder bereits während der Fermentation oder nachträglich angewendet. Das Ziel kann eine Reinigung des Produktes oder eine Trennung verschiedener Produkte voneinander sein. Ein weiteres Ziel ist die Umwandlung des als Salz gewonnenen Produktes in die freie Säure. Die bekannten Verfahren betreffen Itaconsäure (US-A 3 873 425), Milchsäure (DE-A 1 957 395), Gluconsäure und Glutaminsäure. Bei dem Verfahren zur Herstellung der Itaconsäure erfolgt durch Elektrodialyse nur eine Edukt/Produkt-Trennung, und weitere Schritte mit Ionenaustauschern werden erforderlich.

Zur Abtrennung von zum Beispiel L-Äpfelsäure aus dem Reaktionsgemisch werden verschiedene Verfahren beschrieben. So wird vorgeschlagen, L-Äpfelsäure als Ca-Salz zu fällen (DE-A 1 417 033). Dabei wird auch die Fumarsäure gefällt. Beim Abtrennen der Fumarsäure sowie beim Filtrieren sind hier Stoffverluste unvermeidbar. Außerdem werden bei diesem Verfahren fremde Säure und Ca-Verbindungen benötigt. Auch eine Behandlung mit Ionenaustauschern zur Umwandlung des Salzes in freie L-Äpfelsäure wird beschrieben (DE-A 3 247 981). Beim Übergang zum Regenerieren des Ionenaustauschers treten dabei zwangsweise Stoffverluste auf. Nach dem Regenerieren stellt das Eluat eine Umweltbelastung dar. Der Verbrauch einer weiteren Säure zum Regenerieren ist unvermeidlich.

Auch durch Elektrodialyse kann man ein Salz einer organischen Säure BY unter Einsatz einer anderen Säure H₂X in die freie Säure H₂Y überführen:

$$BY + H_2X \rightarrow H_2Y + BX$$

So kann man nach DE-B 1 054 068 eine organische Säure durch Elektrodialyse in einer Elektrodialyseeinheit mit nur Kationentauschermembranen und einer ungeraden Anzahl von Zellen aus ihrem Salz gewinnen. Dabei entsteht ein Salz BX, das entsorgt werden muß.

Die bekannten Verfahren zur Aufarbeitung haben folgende Eigenschaften:

(1) mehrere Verfahrensschritte (Filtrieren, Auswaschen, Dialyse, Ansetzen neuer Lösung)
(2) Verbrauch von systemfremden Stoffen (Ca-Salz, Säuren)
(3) neue für das Verfahren nicht nützliche Stoffströme (Waschwasser, Eluate).

Die Aufgabe der vorliegenden Erfindung besteht darin, ein verbessertes und vereinfachtes Verfahren für eine Elektrodialyse bereitzustellen, bei der das Salz einer organischen Säure in die freie organische Säure übergeführt wird und gleichzeitig das Kation des Salzes der organischen Säure mit dem Anion einer anderen Säure ein Salz bildet. Dabei sollten die Stoffverluste gering sein.

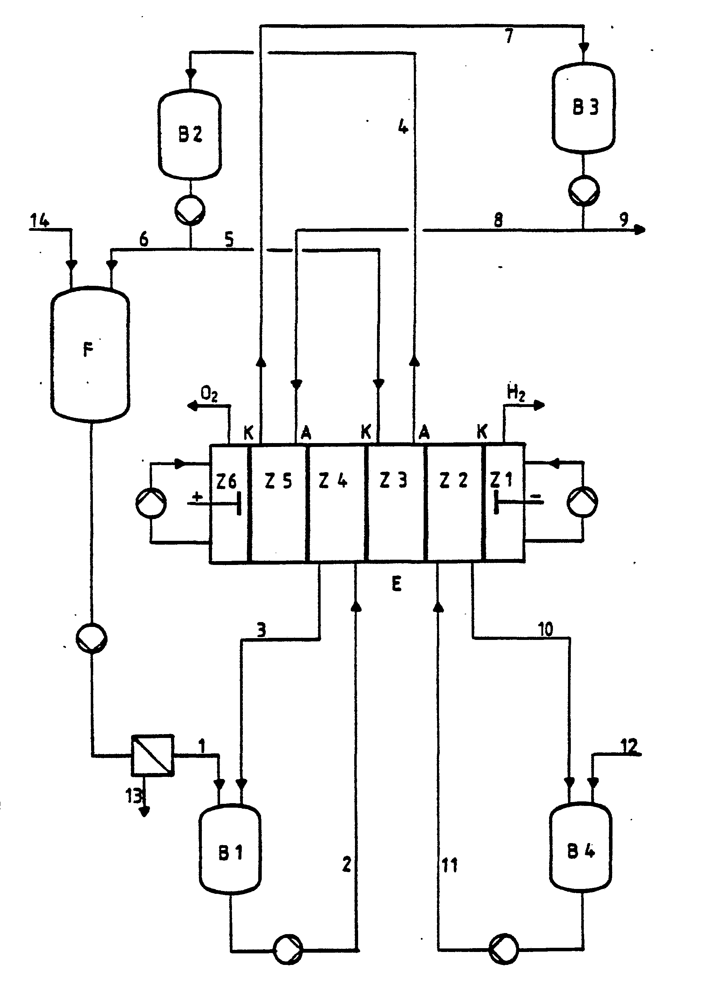

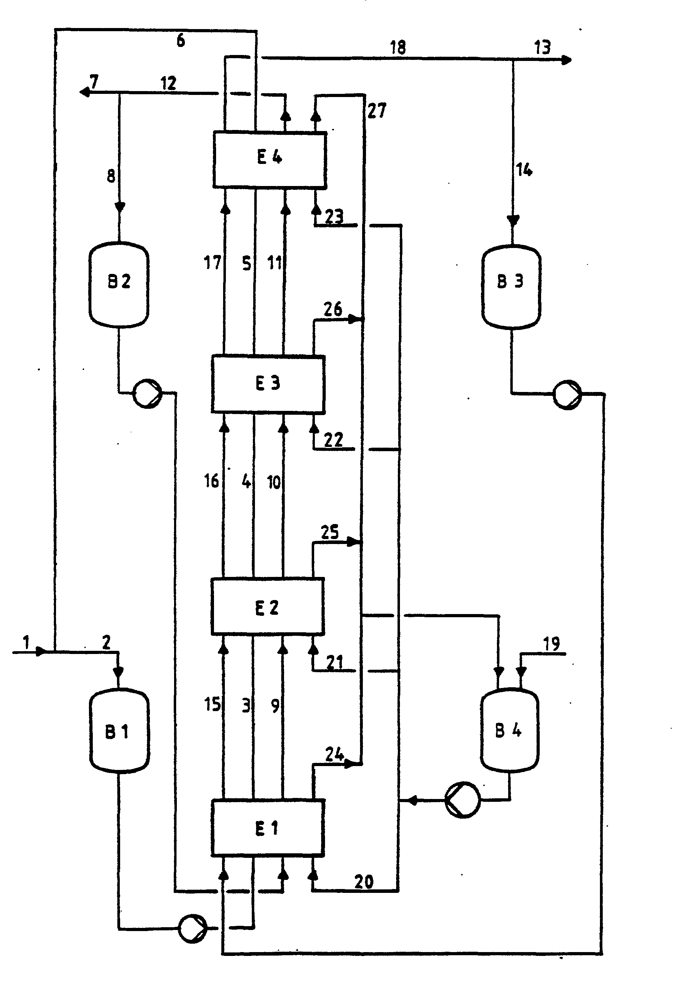

Erfindungsgemäß wird die Aufgabe dadurch gelöst, daß man die Elektrodialyse entsprechend den Abbildungen 1 und 2 in mindestens einer Elektrodialyseeinheit E durchführt, die aus durch Kationentauschermembranen K abgetrennten Kathodenzelle Z1 und Anodenzelle Z6 sowie aus 4 zwischengeschalteten Dialysezellen (Z2 bis Z5) bestehen, die abwechselnd durch Kationen- und Anionentauschermembranen (K, A) voneinander getrennt sind. Dabei können Anordnungen gewählt werden, bei denen die Zellen Z2 bis Z5 mehrfach wiederholt werden, so daß bei Verdopplung 8 Zellen und 2 Elektrodenzellen und bei Verdreifachung 12 Zellen und 2 Elektrodenzellen vorliegen.

Während der Elektrodialyse wird das Salz der organischen Säure durch mindestens eine Zelle Z4 und die andere Säure durch mindestens eine Zelle Z2 geleitet. Die freie organische Säure gewinnt man dabei aus einem Produktstrom von mindestens einer Zelle Z5 nach bekannten Methoden.

Die Elektrodialyse wird vorzugsweise bei 30 bis 100°C durchgeführt, wobei Temperaturen von 50 bis 60°C besonders bevorzugt werden.

In einer bevorzugten Ausführungsform wird die Elektrodialyse in einer Reihe von 2 bis 10 Elektrodialyseeinheiten durchgeführt. Falls eine der Säuren, wie beispielsweise Fumarsäure, in Wasser schwerlöslich ist, wird die Säurelösung in parallelen Strömen durch die Elektrodialyseeinheiten geleitet. Die anderen Lösungen, wie zum Beispiel Fumarat-, Malat- und L-Äpfelsäurelösung, durchlaufen die einzelnen Elektrodialyseeinheiten nacheinander in Serie.

Vorzugsweise schließt man die Elektrodialyse mit einer Fermentation, in der das Salz der organischen Säure gewonnen wird, zu einem Verfahren zusammen. Für den Fall der Gewinnung von L-Äpfelsäure kann man dabei in wäßriger·Lösung sowohl Fumarsäure in Fumarat, das fermentativ in Malat umgewandelt wird, als auch Malat in L-Äpfelsäure überführen. Dabei finden die beiden elektrodialytischen Umwandlungen Fumarsäure/Fumarat und Malat/L-Äpfelsäure bei 30 bis 100°C zur selben Zeit im selben Elektrodialysegerät statt.

Die in Wasser schwer lösliche Fumarsäure wird vorzugsweise in ein leichtlösliches Alkali- oder Ammoniumfumarat übergeführt, das als eigentliches Substrat für die Fermentation eingesetzt wird. Bei der Fermentation entsteht dann aus dem Fumarat das entsprechende Alkali- oder Ammoniummalat.

Bei kontinuierlicher Fermentation erfolgt dabei eine kontinuierliche Elektrodialyse. Bei diskontinuierlicher Fermentation wird auch die Elektrodialyse diskontinuierlich ausgeführt. Dabei werden beispielsweise bei der L-Äpfelsäureherstellung bei diskontinuierlicher Fahrweise Zwischenbehälter zum Sammeln der Fumarat- und der Malatlösung benötigt.

Bei kontinuierlicher und diskontinuierlicher Fahrweise werden Fumarsäure-, Fumarat-, Malat- und L-Äpfelsäurelösung im Kreise geführt. Bei diskontinuierlicher Fahrweise wird die Elektrodialyse beendet, wenn die L-Äpfelsäurelösung 10 bis 70% L-Äpfelsäure enthält. Bei kontinuierlicher Fahrweise wird eine L-Äpfelsäurelösung mit 10 bis 70% L-Äpfelsäure im Kreise geführt. Aus diesen L-Äpfelsäurelösungen kann durch Konzentrierung, Abkühlung und Kristallisation reine L-Äpfelsäure hergestellt werden.

Die organischen Säuren, die nach dem vorliegenden Verfahren gewonnen werden können, enthalten vorzugsweise 3 bis 8 C-Atome und bis zu 3 Carboxylgruppen und gegebenenfalls eine oder mehrere Hydroxyl-, Amino- und Ketogruppen. Beispiele für diese organischen Säuren sind Äpfel-, Asparagin-, Itacon-, Milch-, Wein-, Glucon-, Glutamin- und Adipinsäure.

Eine Besonderheit der vorliegenden Erfindung besteht darin, daß durch die Kopplung der Substrat- und Produktströme bei der Elektrodialyse ein geschlossener Kreislauf gebildet wird. Die Elektrodialyse dient nicht nur in der Aufarbeitungsstufe zur Freisetzung der organischen Säure, sondern auch, wenn beispielsweise Malat fermentativ aus Fumarat hergestellt wird, in der Vorstufe zur Bereitstellung von Fumaratlösungen für die Fermentation. Überraschenderweise kann dann die schwerlösliche Fumarsäure mittels Elektrodialyse unmittelbar der Fermentation verfügbar gemacht werden. Eine vorgeschaltete Reaktionsstufe, in der Fumarsäure mit einer Lauge umgesetzt wird, ist nicht erforderlich.

Außer auf Laugen wird auch auf Mineralsäuren (zum Freisetzen der organischen Säure) verzichtet. Es entstehen weder fremde Stoffe noch Abwässer. Stoffverluste sind deshalb sehr gering.

Abbildung 1 zeigt als Beispiel ein Schema für die fermentative Herstellung von L-Äpfelsäure und die Aufarbeitung durch Elektrodialyse. Die Elektrodialyseeinheit E besteht aus 6 Zellen, aus Kathoden- und Anodenzelle (Z1 und Z6) und 4 weiteren Zellen (Z2 bis Z5), die durch Kationentauschermembranen K oder durch Anionentauschermembranen A voneinander getrennt sind. Durch Z1 und Z6 wird eine verdünnte Schwefelsäure geleitet.

Durch Z2 und Behälter B4 und über die Ströme 10 und 11 wird eine Fumarsäurelösung im Kreise geleitet. Durch Z3 und Behälter B2 und über die Ströme 4 und 5 wird eine Ammoniumfumaratlösung im Kreise geführt. Durch Z4 und Behälter B1 und über die Ströme 3 und 2 wird eine Ammoniummalatlösung gepumpt. Eine L-Äpfelsäurelösung wird durch Z5 und Behälter B3 und über die Ströme 7 und 8 geleitet.

Fumarsäure und Wasser werden über Strom 12 eingeführt. L-Äpfelsäure wird über Strom 9 gewonnen.

Wird die Fermentation diskontinuierlich betrieben, so wird aus dem Vorratsbehälter B2 Ammoniumfumaratlösung über Strom 6 in den Fermenter F geleitet. Über Strom 14 werden Zellmasse, Nährlösung und weitere Fermentationshilfsmittel in den Fermenter eingeführt.

Der Ablauf aus dem Fermenter besteht aus einer Ammoniummalatlösung, die nach Abtrennung von Zellmasse (Strom 13) über Strom 1 in den Behälter B1 geleitet und dort gesammelt wird. Zeitlich unabhängig von der Fermentation erfolgt dann die Elektrodialyse ebenfalls diskontinuierlich.

Bei kontinuierlicher Fermentation wird eine kontinuierliche Elektrodialyse durchgeführt. Ammoniumfumarat wird dabei über Strom 6 aus dem Elektrodialysekreislauf für die Fermentation abgezweigt. Über Strom 1 wird Ammoniummalat in den Kreislauf eingespeist.

In der Elektrodialyseeinheit wandern beim Anlegen eines elektrischen Feldes Protonen von Z2 nach Z1, Fumarationen von Z2 nach Z3, Ammoniumionen von Z4 nach Z3, Malationen von Z4 nach Z5 und Protonen von Z6 nach Z5. Auf diese Weise wird L-Äpfelsäure in Z5 gebildet und dort angereichert.

Über den Produktstrom 9 wird die L-Äpfelsäure gewonnen. Die beim Eindampfen der L-Äpfelsäurelösung möglicherweise anfallenden Verunreinigungen an Fumarsäure können über Strom 12 in den Prozeß zurückgeführt werden.

Abbildung 2 zeigt für den gleichen Fall der fermentativen Herstellung von L-Äpfelsäure aus Fumarsäure als Beispiel schematisch die Elektrodialyse in 4 Elektrodialyseeinheiten E1 bis E4. Wegen der geringen Löslichkeit der Fumarsäure in Wasser ist es zweckmäßig, daß man die Fumarsäurelösung aus dem Behälter B4 über 4 Parallelströme 20, 21, 22 und 23 durch die einzelnen Einheiten leitet. Die Lösungen von Ammoniumfumarat (Ströme 9 bis 12), Ammoniummalat (Ströme 3 bis 6) und L-Äpfelsäure (Ströme 15 bis 18) durchlaufen die 4 Einheiten nacheinander.

Über Strom 19 wird Fumarsäure zugegeben. Über Strom 7 wird Fumarat dem Fermenter und über Strom 1 Malat der Elektrodialyse zugeführt.

Aus dem Produktstrom 13 wird L-Äpfelsäure gewonnen.

Beispiel 1

Für die Elektrodialyse wurde eine von Zellmasse befreite Ammoniummalatlösung eingesetzt, die mit Hilfe des Pilzes Aspergillus wentii aus Ammoniumfumaratlösung hergestellt worden war.

Die Elektrodialyse wurde in einer Elektrodialyseeinheit mit 8 Zellen und 2 Elektrodenkammern durchgeführt. Die Kationen- und Anionentauschermembranen waren handelsübliche Produkte auf der Basis von Styrol-Divinylbenzol-Copolymeren.

| | |
|---|---|
| Membranfläche | 1 dm² |
| Dicke der Membranen | 0,2 mm |
| Abstand zwischen den Membranen | 2 mm |

Zum Versuchsbeginn wurden folgende Lösungen vorgelegt:

| Elektrodenkammern: | 5%ige Schwefelsäure | | |
|---|---|---|---|
| Fumarsäurekreislauf: | Fumarsäure | 80 | g |
| | Wasser | 3 815 | g |
| Ammoniumfumaratkreislauf: | Wasser | 3 042 | g |
| Ammoniummalatkreislauf: | Ammoniummalat | 1 060 | g |
| | Ammoniumfumarat | 150,4 | g |
| | Wasser | 3 262,7 | g |
| L-Äpfelsäurekreislauf: | L-Äpfelsäure | 188 | g |
| | Fumarsäure | 2,5 | g |
| | Wasser | 1 079,5 | g |

Die Elektrodialyse erfolgte bei 60°C bei einem Volumenstrom von 140 l/h bei jedem Kreislauf und einer Potentialdifferenz von 8V. Die Stromstärke betrug 1,05 bis 1,25 A.

Nach 8 Stunden wurden 40 g Fumarsäure dem Behälter B4 zugegeben. Der Versuch dauerte insgesamt 12 Stunden.

Der Fumaratkreislauf enthielt am Ende des Versuchs 3,8 Gew.-% Ammoniumfumarat.

Die Produktlösung (L-Äpfelsäurekreislauf) hatte die folgende Zusammensetzung:

| | | | |
|---|---|---|---|
| L-Äpfelsäure | 246,4 g | 15 | Gew.-% |
| Fumarsäure | 19,4 g | 1,2 | Gew.-% |
| Ammoniummalat | 0,3 g | 180 | Gew.-ppm |
| Ammoniumfumarat | 0,4 g | 240 | Gew.-ppm |
| Wasser | 1 378,0 g | 83,8 | Gew.-% |

Es wurden 58,4 g Äpfelsäure hergestellt bzw. 50,6 g Fumarsäure umgesetzt. Eine Bildung von Bernsteinsäure durch evtl. Reduktion der Fumarsäure durch Wasserstoff konnte ausgeschlossen werden. Der Schwefelgehalt lag unter 20 ppm.

Beim Abkühlen der Produktlösung auf Raumtemperatur fielen Kristalle (92 Gew.-% Fumarsäure, 8 Gew.-% L-Äpfelsäure) aus, die abgetrennt wurden. Anschließend wurden bei 50 mbar und 50°C 1260 ml Wasser destilliert. Beim Abkühlen der konzentrierten Produktlösung auf Raumtemperatur fielen Kristalle folgender Zusammensetzung an:

95,3 Gew.-% L-Äpfelsäure
4,7 Gew.-% Fumarsäure

Ein weiteres Einengen der Mutterlauge bis zur Trockne ergab Kristalle der Zusammensetzung:
98,3 Gew.-% L-Äpfelsäure
1,7 Gew.-% Fumarsäure

Beispiel 2

Auf bekannte Weise (aus Ammoniumfumarat mittels Aspartase) hergestellte 20%ige Ammoniumasparagatlösung wurde durch Filtration und Zentrifugation von hochmolekularen und kolloidalen Teilen befreit. Die Lösung wurde gemäß Beispiel 1 bei 50°C mit Fumarsäure umgesetzt. Wegen der geringen Löslichkeit der freien Asparaginsäure wurde der entsprechende Kreislauf über ein Filter geleitet und die ausfallende Säure abgefangen. Dabei wurde wie auch in den folgenden Beispielen die gleiche Versuchsapparatur wie in Beispiel 1 eingesetzt. Anfangskonzentration im

**Asparagatkreislauf:**          1 012 g Ammoniumasparagat

                                 4 020 g Wasser und sonstige Stoffe

**Fumaratkreislauf:**            980 g Ammoniumfumarat

                                 3 870 g Wasser

**Fumarsäurekreislauf:**         gesättigte Fumarsäure (< 2 Gew.-%)

**Asparaginsäurekreislauf:**     Wasser

Es entstanden 55 g Asparaginsäure. Entsprechend wurde eine Bildung von 63 g Ammoniumfumarat beobachtet.

Beispiel 3

Auf bekannte Weise fermentativ hergestellte Itaconsäure (vgl. US-A 3 873 425) wurde gemäß Beispiel 1 zunächst von störenden Komponenten befreit und mittels Ionenaustauscher in Kaliumitaconat umgewandelt. Das Kaliumsalz wurde dann unmittelbar mit Schwefelsäure erfindungsgemäß elektrodialytisch umgesetzt:

$$K_2 \, It + H_2SO_4 \rightarrow K_2SO_4 + H_2 \, It$$
$$(It = Itaconat)$$

Die eingesetzte Kaliumitaconatlösung war 4%ig und wurde mit 5%iger $H_2SO_4$ umgesetzt. Aus 104 g Kaliumitaconat entstanden 64 g Itaconsäure neben 86 g $K_2SO_4$.

Beispiel 4

Eine 10%ige Natriumlactatlösung wurde mit einer im Sättigungszustand gehaltenen Lösung von $CO_2$ in Wasser erfindungsgemäß bei 30°C umgesetzt. Aus 46 g Natriumlactat entstanden 36 g Milchsäure und 20 g Natriumcarbonat, das als Lösung zum Beispiel zur Neutralisation und pH-Regulierung einer Fermentation eingesetzt werden kann. Eine Weiterreinigung der Milchsäure kann nach bekannten Methoden durch Ionenaustauscher, Veresterung oder Extraktion erfolgen.

Beispiel 5

Eine 12%ige Natriumtartratlösung wurde erfindungsgemäß bei 30°C mit 10%iger Schwefelsäure einer Umsetzung unterworfen. Aus 85 g Natriumtartrat wurden 65 g Weinsäure erhalten. Außerdem fielen 62 g Natriumsulfat an.

Beispiel 6

90 g Natriumgluconat wurden als 12%ige Lösung wie in Beispiel 5, jedoch bei 60°C, mit Schwefelsäure umgesetzt. Dabei entstanden 78 g Gluconsäure und die entsprechende Menge Natriumsulfat.

Beispiel 7

72 g Natriumglutaminat wurden als 10%ige Lösung mit Schwefelsäure wie in Beispiel 6 erfindungsgemäß zu 54 g Glutaminsäure und der entsprechenden Menge Natriumsulfat umgesetzt.

Beispiel 8

85 g Natriumadipat wurden als 10%ige Lösung nach Filtration wie in Beispiel 2 analog zu Beispiel 6 mit Schwefelsäure zu 64 g Adipinsäure und Natriumsulfat umgesetzt.

**Patentansprüche**

1. Verfahren zur Gewinnung einer freien organischen Säure aus einem Salz dieser Säure durch Elektrodialyse, bei der das Salz der organischen Säure in die freie organische Säure übergeführt wird und gleichzeitig das Kation des Salzes der organischen Säure mit dem Anion einer anderen Säure ein Salz bildet, wobei beide Umwandlungen in ein- und derselben Elektrodialyseeinheit ausgeführt werden, dadurch gekennzeichnet, daß man die Elektrodialyse in mindestens einer Einheit (E) durchführt, die aus durch Kationenaustauschermembranen (K) abgetrennten Kathoden- und Anodenzellen (Z1, Z6) sowie n zwischengeschalteten Dialysezellen (Z2 bis Z5) besteht, die abwechselnd durch Kationen- und Anionenaustauschermembranen (K, A) voneinander getrennt sind und wobei n 4 oder ein Mehrfaches von 4 ist, und daß man während der Elektrodialyse das Salz der organischen Säure durch mindestens eine Zelle (Z4) und die andere Säure durch mindestens eine Zelle (Z2) leitet und daß man die freie organische Säure aus einem Produktstrom von mindestens einer Zelle (Z5) nach bekannten Methoden gewinnt.

2. Verfahren nach Anspruch 1, dadurch gekennzeichnet, daß die Elektrodialyse bei 30 bis 100°C, vorzugsweise bei 50 bis 60°C, durchgeführt wird.

3. Verfahren nach Anspruch 1, dadurch gekennzeichnet, daß man die Elektrodialyse in 2 bis 10 Elektrodialyseeinheiten durchführt, wobei die Ströme einer schwerlöslichen Säure parallel und die übrigen Ströme nacheinander in Serie durch diese Einheiten fließen.

4. Verfahren nach Anspruch 1, dadurch gekennzeichnet, daß das Salz der organischen Säure durch Fermentation gewonnen wird und bei diskontinuierlicher Fermentation die Elektrodialyse diskontinuierlich erfolgt.

5. Verfahren nach Anspruch 1, dadurch gekennzeichnet, daß das Salz der organischen Säure durch Fermentation gewonnen wird und bei kontinuierlicher Fermentation die Elektrodialyse kontinuierlich erfolgt.

6. Verfahren nach den Ansprüchen 1 bis 5, dadurch gekennzeichnet, daß die organische Säure 3 bis 8 C-Atome enthält und bis zu 3 Carboxylgruppen und gegebenenfalls eine oder mehrere Hydroxyl-, Amino- und/oder Ketogruppen aufweist.

7. Verfahren nach Anspruch 6, dadurch gekennzeichnet, daß die organische Säure Äpfelsäure, Asparaginsäure, Itaconsäure, Milchsäure, Weinsäure, Gluconsäure, Glutaminsäure oder Adipinsäure ist.

8. Verfahren nach den Ansprüchen 1 bis 7, dadurch gekennzeichnet, daß fermentativ hergestelltes Malat in L-Äpfelsäure übergeführt wird und aus Fumarsäure Fumarat gebildet wird, wobei beide elektrodialytischen Umwandlungen bei 30 bis 100°C ausgeführt werden.

9. Verfahren nach Anspruch 8, dadurch gekennzeichnet, daß das gebildete Fumarat ein Alkali- oder Ammoniumfumarat ist und als Malat ein Alkali- oder Ammoniummalat verwendet wird.

10. Verfahren nach Anspruch 8, dadurch gekennzeichnet, daß man eine wäßrige L-Äpfelsäurelösung im Kreise führt und die Elektrodialyse beendet, wenn diese Lösung 10 bis 70% L-Äpfelsäure enthält.

**Claims**

1. A process for obtaining a free organic acid from a salt of this acid by electrodialysis, in which the salt of the organic acid is converted into the free organic acid and simultaneously the cation of the salt of the organic acid forms a salt with the anion of another acid, both conversions being carried out in one and the same electrodialysis unit, characterized in that the electrodialysis is carried out in at least one unit (E) which consists of cathode and anode cells (Z1, Z6) separated by cation exchanger membranes (K), and of n interposed dialysis cells (Z2 to Z5), which are alternately separated from one another by cation and anion exchanger membranes (K, A), being 4 or a multiple of 4, and in that, during the electrodialysis, the salt of the organic acid is passed through at least one cell (Z4) and the other acid is passed through at least one cell (Z2), and in that the free organic acid is obtained by known methods from a production stream of at least one cell (Z5).

2. A process according to claim 1, characterized in that the electrodialysis is carried out at 30 to 100°C, preferably 50 to 60°C.

3. A process according to claim 1, characterized in that the electrodialysis is carried out in 2 to 10 electrodialysis units, the streams of a sparingly soluble acid flowing in parallel and the other streams flowing consecutively in series through these units.

4. A process according to claim 1, characterized in that the salt of the organic acid is obtained by fermentation, and the electrodialysis is carried out batchwise with batchwise fermentation.

5. A process according to claim 1, characterized in that the salt of the organic acid is obtained by fermentation, and the electrodialysis is carried out continuously with continuous fermentation.

6. A process according to any of claims 1 to 5, characterized in that the organic acid contains 3 to 8 C atoms and up to 3 carboxyl groups and optionally has one or more hydroxyl, amino and/or keto groups.

7. A process according to claim 6, characterized in that the organic acid is malic acid, aspartic acid, itaconic acid, lactic acid, tartaric acid, gluconic acid, glutamic acid or adipic acid.

8. A process according to any of claims 1 to 7, characterized in that malate prepared by fermentation is converted into L-malic acid and fumarate is formed from fumaric acid, both electrodialytic conversions being carried out at 30 to 100°C.

9. A process according to claim 8, characterized in that the fumarate formed is an alkali metal fumarate or ammonium fumarate, and, as malate, an alkali metal malate or ammonium malate is used.

10. A process according to claim 8, characterized in that an aqueous L-malic acid solution is circulated, and the electrodialysis is terminated when this solution contains 10 to 70% of L-malic acid.

**Revendications**

1. Procédé pour obtenir à l'état libre un acide organique à partir d'un sel de cet acide, par une électrodialyse lors de laquelle le sel de l'acide organique est transformé en l'acide organique libre et lors de laquelle simultanément le cation du sel de l'acide organique forme un sel avec l'anion d'un autre acide, tandis que les deux transformations sont effectuées dans une seule et même unité d'électrodialyse, caractérisé par le fait que l'électrodialyse est effectuée dans au moins une unité (E) qui est constituée par des cellules cathodiques et anodiques (Z1, Z6) séparées par des membranes (K) échangeuses de cations, ainsi que par $\underline{n}$ cellules intermédiaires de dialyse (Z2 à Z5) qui sont alternativement séparées l'une de l'autre par des membranes (K, A) échangeuses de cations et d'anions, $\underline{n}$ étant égal à 4 ou à un multiple de 4, et par le fait que pendant l'électrodialyse le sel de l'acide organique passe par au moins une cellule (Z4) et que l'autre acide passe par au moins une cellule (Z2) et que l'on obtient l'acide organique à l'état libre selon des méthodes connues à partir d'un courant de produit provenant d'au moins une cellule (Z5).

2. Procédé selon la revendication 1, caractérisé par le fait que l'électrodialyse est effectuée à une température de 30 à 100°C, de préférence à une température de 50 à 60°C.

3. Procédé selon la revendication 1, caractérisé par le fait que l'on effectue l'électrodialyse dans 2 à 10 unités d'électrodialyse, les courants d'un acide difficilement soluble s'écoulant parallèlement et les autres courants l'un après l'autre en série à travers ces unités.

4. Procédé selon la revendication 1, caractérisé par le fait que le sel de l'acide organique est obtenu par fermentation et que, dans le cas d'une fermentation discontinue, l'électrodialyse a lieu de façon discontinue.

5. Procédé selon la revendication 1, caractérisé par le fait que le sel de l'acide organique est obtenu par fermentation et que, dans le cas d'une fermentation continue, l'électrodialyse a lieu de façon continue.

6. Procédé selon les revendications 1 à 5, caractérisé par le fait que l'acide organique renferme de 3 à 8 atomes de carbone et jusqu'à trois groupes carboxyle et, le cas échéant, un ou plusieurs groupes hydroxyle, amine et/ou cétone.

7. Procédé selon la revendication 6, caractérisé par le fait que l'acide organique est l'acide malique, l'acide aspartique, l'acide itaconique, l'acide lactique, l'acide tartrique, l'acide gluconique, l'acide glutamique ou l'acide adipique.

8. Procédé selon les revendications 1 à 7, caractérisé par le fait que du malate préparé par fermentation est transformé en acide L-malique et qu'à partir d'acide fumarique, on forme le fumarate, les deux transformations par électrodialyse étant effectuées à une température de 30 à 100°C.

9. Procédé selon la revendication 8, caractérisé par le fait que le fumarate formé est un fumarate de métal alcalin ou d'ammonium et qu'on utilise, comme malate, un malate de métal alcalin ou d'ammonium.

10. Procédé selon la revendication 8, caractérisé par le fait que l'on fait circuler une solution aqueuse d'acide L-malique et que l'on termine l'électrodialyse lorsque cette solution renferme de 10 à 70 % d'acide L-malique.